# EUROPEAN PATENT SPECIFICATION

(11) **EP 3 949 877 B1**
(45) Date of publication and mention of the grant of the patent: **15.01.2025**
(21) Application number: 20781299.1
(22) Date of filing: 31.03.2020
(51) Int. Cl.: A61B 17/128, A61B 17/00

(54) **ACTUATION STRUCTURE OF BLOOD VESSEL CLIP APPLIER**
BETÄTIGUNGSSTRUKTUR EINES BLUTGEFÄSSKLAMMERAPPLIKATORS
STRUCTURE D'ACTIONNEMENT D'APPLICATEUR D'AGRAFES POUR VAISSEAU SANGUIN

(30) Priority: 03.04.2019 CN 201910266128
(43) Date of publication of application: 09.02.2022
(73) Proprietor: MEDSCOPE BIOTECH CO., LTD., Taiwan 350 (TW)
(72) Inventor: HUANG, Shin-Hao, Hsinchu County Taiwan (CN); WU, Chen-Xuan, Hsinchu City 300 Taiwan (CN)
(74) Representative: Straus, Alexander
(86) International application number: PCT/CN2020/082541
(87) International publication number: WO 2020/200211

(56) References cited:
- EP-A1- 3 066 995
- CN-A- 105 286 936
- CN-A- 107 595 352
- CN-A- 108 065 979
- US-A- 5 972 003
- US-A- 6 099 537
- US-A1- 2007 185 504

## Description

### BACKGROUND OF THE INVENTION

### 1. Field of the Invention

The present invention is related to blood vessel clip applier technology, especially an actuation structure of a blood vessel clip applier.

### 2. Description of the Related Art

The known actuation structure of a blood vessel clip applier has a more complicated transmission mechanism, so its transmission is less reliable, and may cause overlapping actions or neutral gears. EP 3 066 995 A1 discloses a surgical clip applier including a handle assembly and an endoscopic assembly. The handle assembly comprises a housing, a trigger pivotally supported on and extending from the housing, and a drive assembly supported within the housing and operatively actuatable by the trigger. The endoscopic assembly includes a shaft-assembly extending from the housing, the shaft assembly including an outer tube and a pair of jaws supported in and extending from a distal end of the outer tube. US 6 099 537 A relates to a medical treatment instrument exhibiting a treatment section for treating issue, an insertion section which has at one end the treatment section, and an operating section which is provided at the other end of the insertion section, operates the treatment section, and has a fixed portion and a movable portion. The treatment section is actuated by pivoting the movable portion with respect to the fixed portion. The medical treatment instrument is further provided with an actuating main body and a cover. The cover is displaceable between a first position in which the medical treatment instrument is used and a second position in which the inside of the operating section is uncovered. Therefore, the lack of the existing actuation structure of a blood vessel clip applier still needs to be improved.

### SUMMARY OF THE INVENTION

The present invention is defined by the appended independent claim. Further embodiments are defined by the dependent claims.

It is the main object of the present invention to provide an actuation structure of a blood vessel clip applier, which can simplify the transmission mechanism, make the transmission action more reliable, and the action is smoother.

To achieve the object stated above, the present disclosure provides an actuation structure of a blood vessel clip applier driven by an operating unit to make a jaw clamp a clip. The actuation structure of the blood vessel clip applier comprises a body comprising a bump; a tube set connected to the body; a loading push rod movably set in said tube set back and forth for pushing the clip forward; a bolt axially movably arranged on the body; a feeding loop claw pivoted to the bolt, the feeding loop claw comprising a front end and a rear end so configured that when the feeding loop claw moves forward, the feeding loop claw is touched by the bump of the body so that the front end of the feeding loop claw changes from a tilted out position to a pressed down position; a loading plug set in the body to link the loading push rod; a loading spring to push the loading plug to move backward; wherein when the front end of the feeding loop claw is tilted out, the loading plug is pushed forward; when the front end of the feeding loop claw is pressed down and kept away from the loading plug, the loading spring pushes the loading plug back to the original position; a recoil push rod movably set in the tube set, so that after the loading plug returns to the original position, the front end of the feeding loop claw is still depressed to push the recoil push rod forward to drive the jaw clamp the clip; and a recoil spring) used to push the recoil push rod to move backward.

Through the structure and the action process, it can simplify the transmission mechanism, make the transmission action more reliable, and the action is smoother, so that the purpose of the invention is achieved.

Preferably, the bolt is provided with a receiving slot, and the feeding loop claw is installed in the receiving slot.

Preferably, the body comprises a bushing, and the loading plug is installed in the bushing.

Preferably, the actuation structure of the blood vessel clip applier further comprises an elastic piece set on said bolt. The elastic piece can be pressed against the rear end of the feeding loop claw so that the front end of the feeding loop claw is tilted out against the loading plug.

Preferably, the operating unit comprises a bolt link pivoting the bolt, and the pivoting center of the bolt link and the bolt is axially behind the pivoting center of the feeding loop claw and the bolt.

Preferably, the loading plug surrounds the recoil push rod.

### BRIEF DESCRIPTION OF THE DRAWINGS

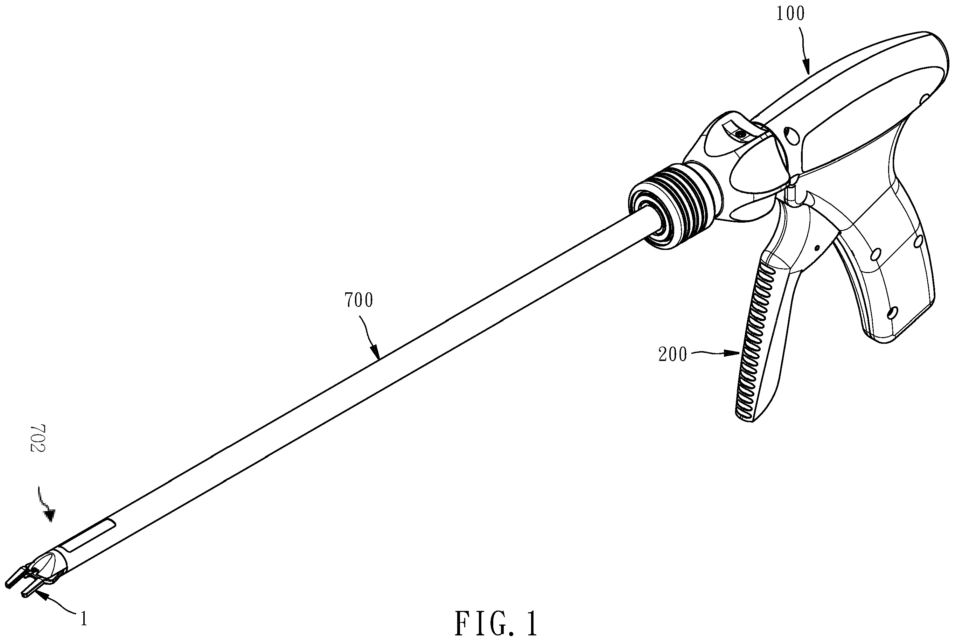

FIG. 1 is an oblique top elevational view of a blood vessel clip applier of the preferred embodiment of the present invention.

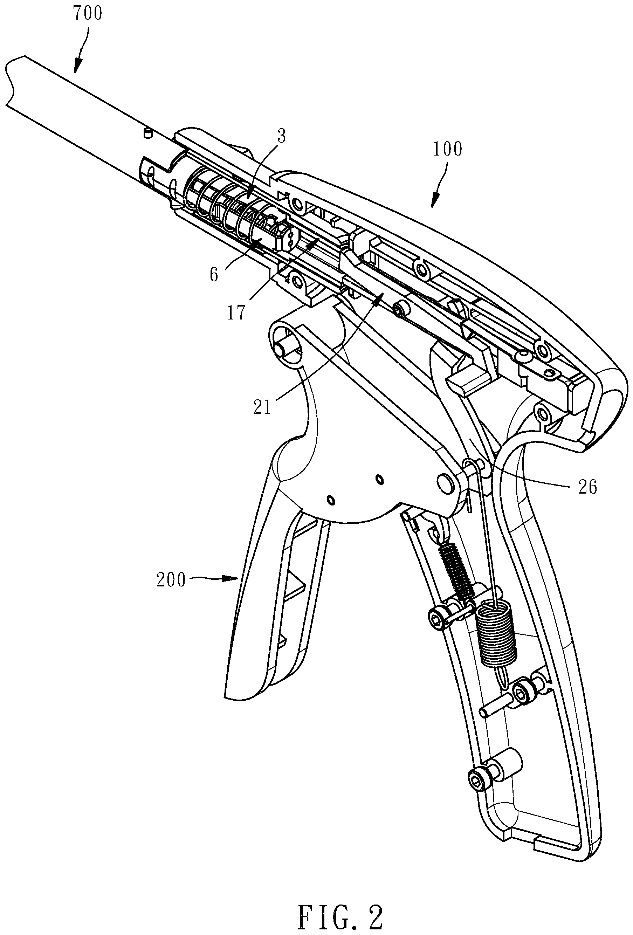

FIG. 2 is a sectional elevational side view of the blood vessel clip applier of the preferred embodiment of the present invention.

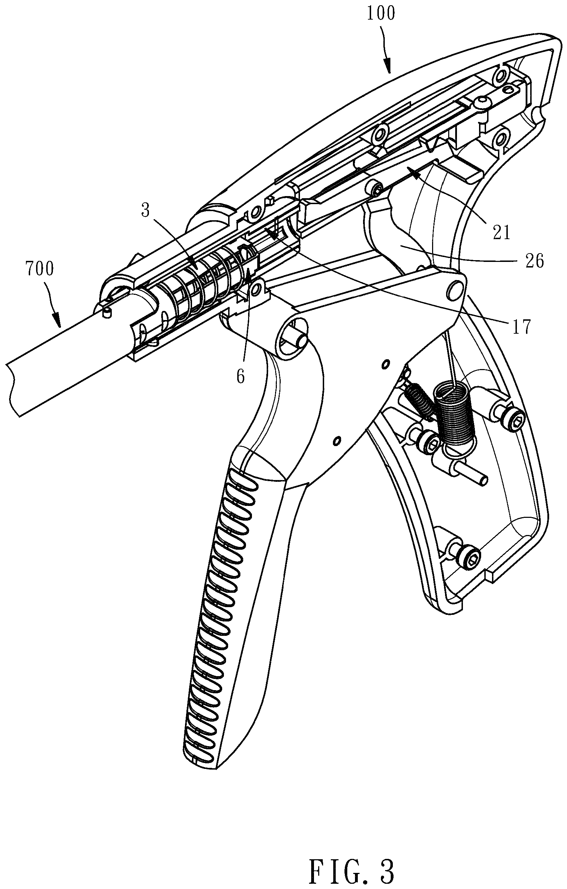

FIG. 3 is another sectional elevational side view of the blood vessel clip applier of the preferred embodiment of the present invention.

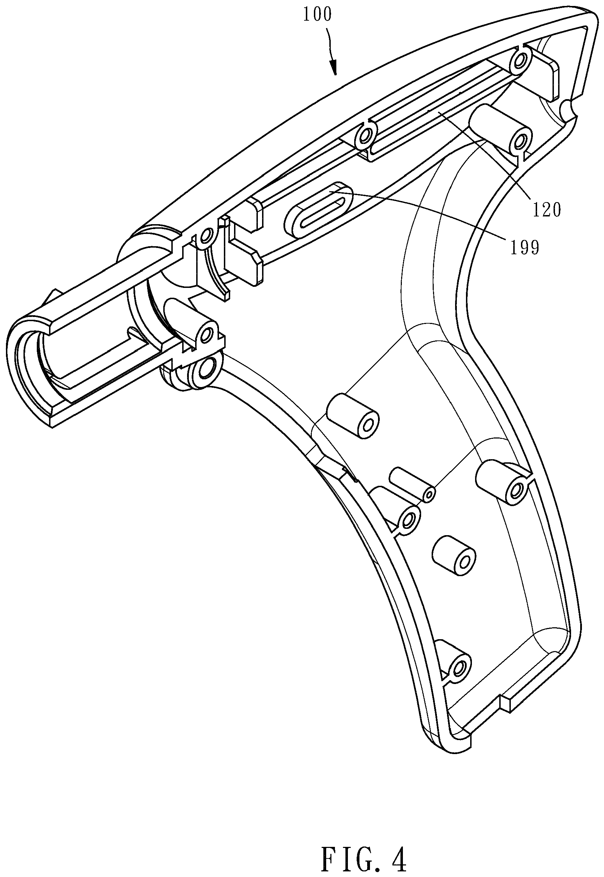

FIG. 4 is a sectional elevational side view of a part of the blood vessel clip applier of the preferred embodiment of the present invention.

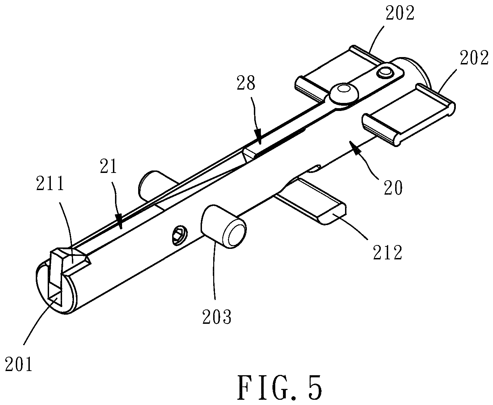

FIG. 5 is an oblique top elevational view of the bolt of the blood vessel clip applier of the preferred embodiment of the present invention.

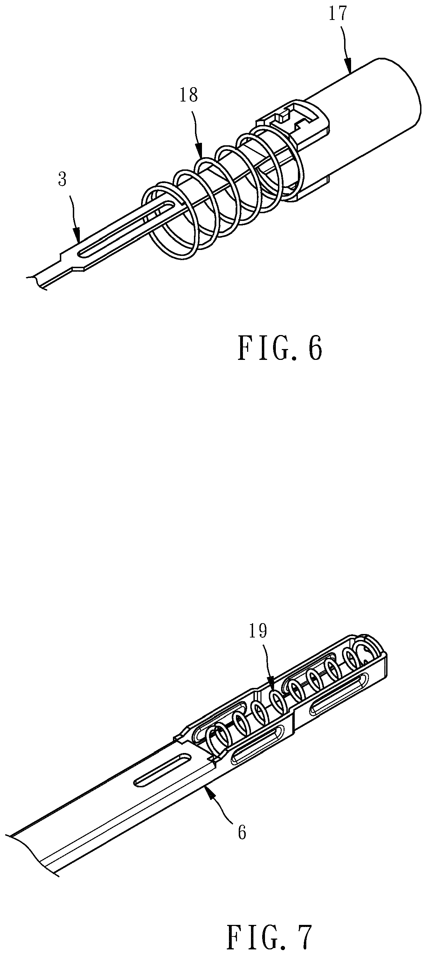

FIG. 6 is an oblique top elevational view of the loading push rod, the loading plug and the loading spring of the bolt of the blood vessel clip applier of the preferred embodiment of the present invention.
FIG. 7 is an oblique top elevational view of the recoil push rod and the recoil spring of the bolt of the blood vessel clip applier of the preferred embodiment of the present invention.
FIG. 8 is an explanatory diagram of the preferred embodiment of the present invention not yet operating state.
FIG. 9 is an explanatory diagram of the preferred embodiment of the present invention just pushed state.
FIG. 10 is a schematic diagram of the preferred embodiment of the present invention, wherein the loading plug has just been pushed back by the loading spring.
FIG. 11 is an explanatory diagram of the preferred embodiment of the present invention where the recoil push rod is pushed forward by the bolt.
FIG. 12 is a three-dimensional exploded view of the front elements of the preferred embodiment of the present invention.

### DETAILED DESCRIPTION OF THE INVENTION

The present invention will be further described below in conjunction with the accompanying drawings and the preferred embodiment:
The actuation structure of a blood vessel clip applier provided by the present invention is driven by an operating unit **200** to make a jaw **1** clamp a clip **35.**

The actuation structure of the blood vessel clip applier comprises:
a body**100** comprising a bushing **110,** a sliding groove **120** located on each of left and right sides of a rear of two shells thereof, and a bump **199** located on each of left and right sides of a middle section of the two shells;
a tube set **700** connected to the body **100;**
a loading push rod **3** movably set on the tube set **700** back and forth to push the clip **35** forward;
a bolt **20** axially movably arranged on the body**100**, the bolt **20** comprising a receiving slot **201** in a front thereof, a sliding wing **202** located on each of left and right sides of a rear thereof and respectively slidingly fitted to the sliding grooves **120** of the body **100,** and a pivot rod **203** located on each of left and right sides of a middle section thereof for the operating unit **200** to pivotally push the bolt **20;**
a feeding loop claw **21** accommodated in the receiving slot **201** of the bolt **20** and pivotally connected to the bolt **20,** in more detail, the feeding loop claw **21** being pivotally connected to the bolt **20** and capable of being tilted to a tilted out position and a pressed down position, the feeding loop claw **21** having a front end **211** and a rear end **212** so configured that when the feeding loop claw **21** moves forward, its rear end **212** is pressed by the bump 199 so that the feeding loop claw **21** reaches the pressed down position;
an elastic piece **28** arranged on the bolt **20,** and the elastic piece **28** being pressable against the rear end **212** of the feeding loop claw **21** so that the front end **211** of the feeding loop claw **21** is lifted out and abuts against a loading plug **17,** in more detail, the elastic piece **28** having a top end that can be pressed against the rear end **212** of the feeding loop claw **21** so that the front end **211** of the feeding loop claw **21** is located at the tilted out position;
wherein, the operating unit **200** has a bolt link **26** pivoting the bolt **20,** and the pivot center of the bolt link **26** and the bolt **20** is axially behind the pivot center of the feeding loop claw **21** and the bolt **20;**
a loading plug **17** installed in the body **100** to link the loading push rod 3; in this embodiment, the loading plug **17** being installed in the bushing **110;**
a loading spring **18** used to push the loading plug **17** and move backward;
wherein when the front end **211** of the feeding loop claw **21** is tilted out, the loading plug **17** can be pushed forward; when the front end **211** of the feeding loop claw **21** is pressed down and kept away from the loading plug **17,** the loading spring **18** can push the loading plug **17** back to the original position;
a recoil push rod **6,** which can be movably set in the tube set **700** back and forth and so configured that after the loading plug **17** returns to the original position, the front end **211** of the feeding loop claw **21** is still depressed and can continue to push the recoil push rod **6** forward to drive the jaw **1** to clamp the clip **35;** and
a recoil spring **19** used to push the recoil push rod **6** and move backward; wherein, the loading plug **17** can surround the recoil push rod **6.**

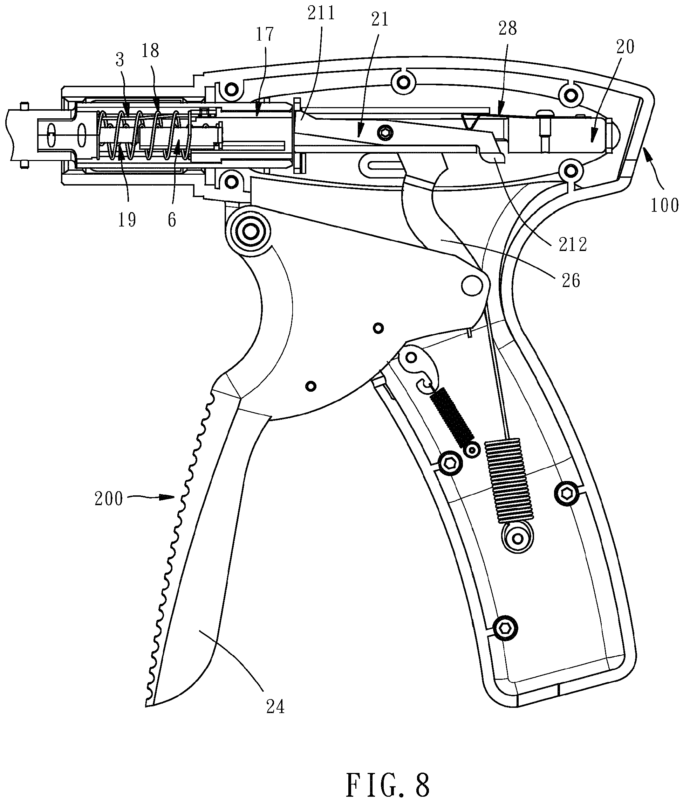

The various operating states of the present invention are described as follows:
As shown in FIG. 8 (original state), where the operating unit **200** has not been operated, the bolt **20** is controlled by the operating unit **200** and is located at the rear of its axis, and the feeding loop claw **21** is pressed to protrude its front end **211,** wherein, the loading plug **17** is pushed by the loading spring **18** and is located at its final position, and the recoil push rod **6** is pushed by the recoil spring **19** and is located at its final position.

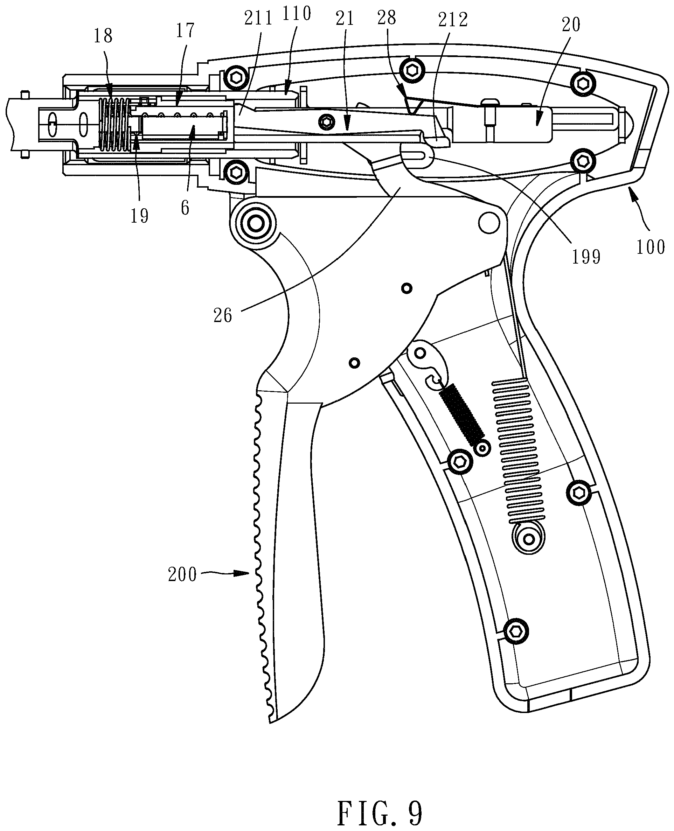

As shown in FIG. 9 (push state), start to operate the operating unit **200,** that is, hold its handle 24, the operating unit **200** drives the bolt **20** to move forward axially, and the front end **211** of the feeding loop claw **21** pushes the loading plug **17** forward (overcomes the elastic resistance of the loading spring **18).**

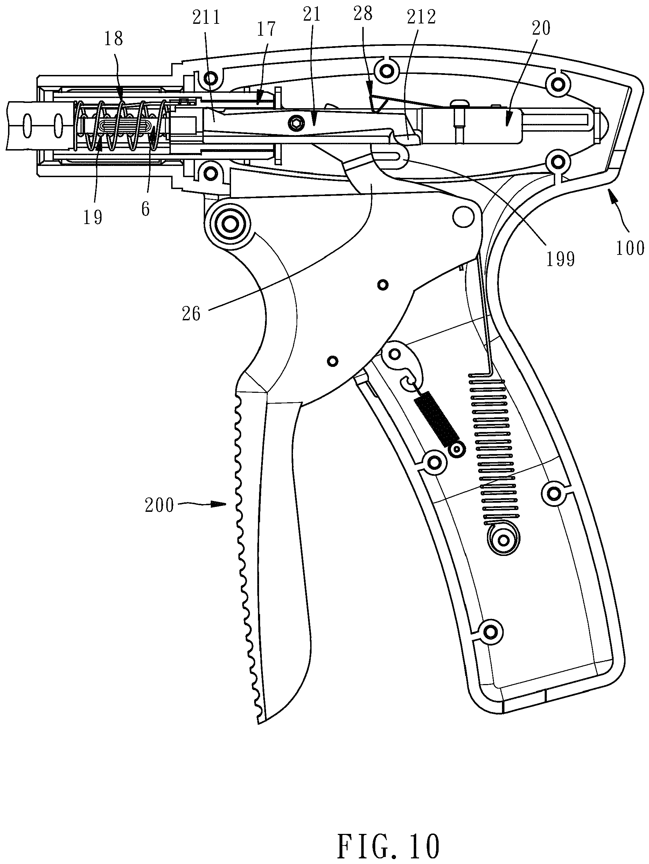

As shown in FIG. 10 (the front end **211** starts to be depressed), when the continuous operation advances, the feeding loop claw **21** deflects because its rear end **212** is touched by the bump **199** of the body **100,** and the front end **211** of the feeding loop claw **21** no longer reaches the loading plug **17,** and the loading plug 17 is then pushed back by the loading spring **18** and returns to its original final position, as shown in FIG. 10, that is, the loading plug **17** has just returned to the state.

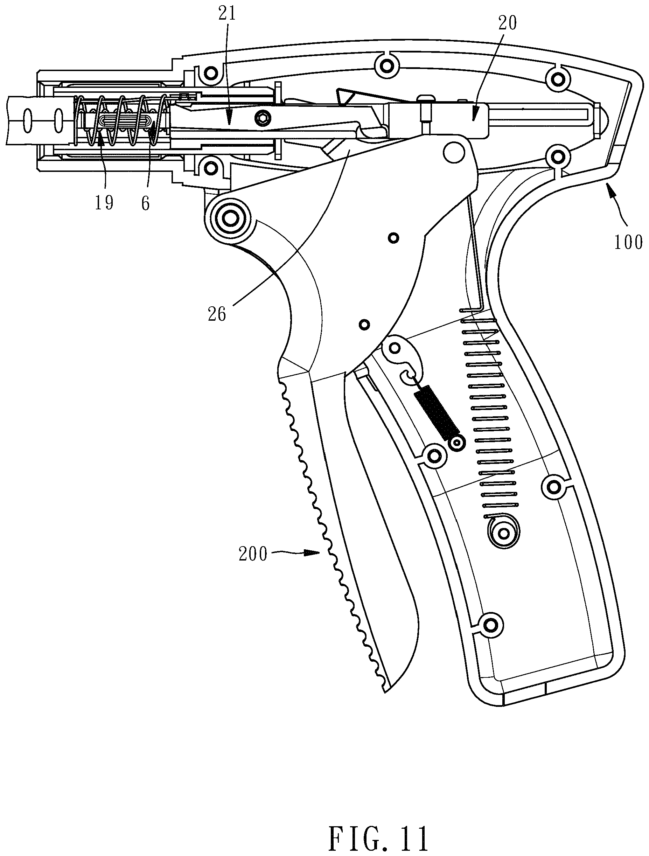

As shown in FIG. 11 (clip 35 clamping state), continue to operate the operating unit **200** again, the bolt **20** is driven by the operating unit **200** to continue to move forward to push the recoil push rod **6 to** overcome the elastic force of the recoil spring **19,** and to make the recoil push rod **6** move forward, so that the recoil push rod **6** can drive the jaw **1** to clamp the clip **35** in it.

Then, release the operating unit **200** without operating, and each component returns to the original state, as shown in FIG. 8.

Through the structure and the action process, it can simplify the transmission mechanism, make the transmission action more reliable, and the action is smoother, so that the purpose of the invention is achieved.

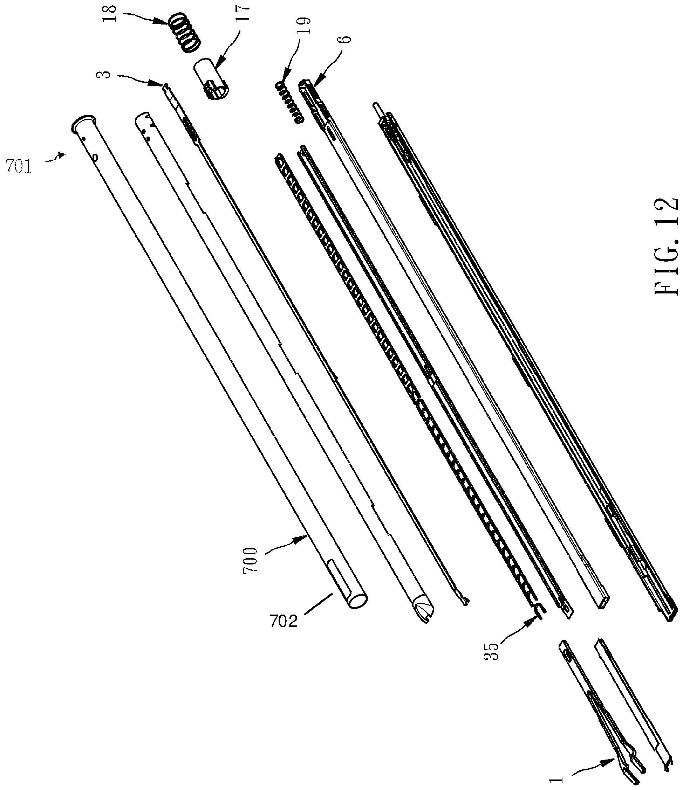

As shown in FIG. 12, a three-dimensional exploded view of the front elements of the preferred embodiment of the present invention is shown, wherein the unexplained structure and principle of action can be generally the same as the invention patent of Taiwan Announcement I627933, which will not be described in detail here. However, the front assembly of the present invention is not limited to only include the structure and principle of action, and can also be of various other types.

Or, the rear end **212** of the feeding loop claw **21** is not necessarily located at the rear, as long as it can be cooperated to activate the feeding loop claw **21.**

In summary, the actuation structure of a blood vessel clip applier of the present invention can indeed simplify the transmission mechanism, make the transmission action more reliable, and the action is smoother, so it does achieve the stated purpose of the invention.

### Industrial applicability:

The actuation structure of a blood vessel clip applier of the present invention can indeed simplify the transmission mechanism, make the transmission action more reliable, and the action is smoother, so it does achieve the purpose of the invention.

Without departing from the scope of the present invention, those skilled in the art can make various corresponding changes and modifications according to the present invention, but these corresponding changes and modifications all should belong to the protection scope of the claims of the present invention.

## Claims

1. An actuation structure of a blood vessel clip applier driven by an operating unit (200) to make a jaw (1) clamp a clip (35), the actuation structure of the blood vessel clip applier comprising:
a body (100) comprising a bump (199);
a tube set (700) comprising a rear end (701) connected to said body (100) and a front end (702) comprising the jaw (1);
a loading push rod (3) movably set in said tube set (700) back and forth for pushing said clip (35) forward to the front end of the tube set (702);
a bolt (20) axially movably arranged on said body (100);
a loading plug (17) set in said body (100) to link said loading push rod (3); **characterized in that** the actuation structure further comprises:
a feeding loop claw (21) pivoted to said bolt (20), said feeding loop claw (21) comprising a front end (211) and a rear end (212) so configured that when said feeding loop claw (21) moves forward in the direction of the rear end of the tube set (701), said feeding loop claw (21) is touched by said bump (199) of said body (100) so that the front end (211) of said feeding loop claw (21) changes from a tilted out position to a pressed down position;
a loading spring (18) to push said loading plug (17) to move backward in the direction of the rear end of the tube set (701);
wherein when the front end (211) of said feeding loop claw (21) is tilted out, said loading plug (17) is pushed forward in the direction of the front end of the tube set (702); wherein when the front end (211) of said feeding loop claw (21) is pressed down the front end (211) is kept away from said loading plug (17), so that said loading spring (18) pushes said loading plug (17) back to its original position;
a recoil push rod (6) movably set in said tube set (700), so that after said loading plug (17) returns to its original position, the front end (211) of said feeding loop claw (21) is still pressed down to push said recoil push rod (6) forward in the direction of the front end of the tube set (702) to drive said jaw (1) clamp said clip (35); and
a recoil spring (19) used to push said recoil push rod (6) to move backward in a direction of the rear end of the the tube set (701).

2. The actuation structure of the blood vessel clip applier as claimed in claim 1, wherein said bolt (20) comprises a receiving slot (201), and said feeding loop claw (21) is installed in said receiving slot (201).

3. The actuation structure of the blood vessel clip applier as claimed in claim 1 or 2, wherein said body (100) comprises a bushing (110), and said loading plug (17) is installed in said bushing (110).

4. The actuation structure of the blood vessel clip applier as claimed in claim 1, 2 or 3, further comprising an elastic piece (28) set on said bolt (20), said elastic piece (28) being pressable against the rear end (212) of said feeding loop claw (21) so that the front end (211) of said feeding loop claw (21) is tilted out against said loading plug (17).

5. The actuation structure of the blood vessel clip applier as claimed in claim 1, 2, 3 or 4, wherein said operating unit (200) comprises a bolt link (26) pivoting said bolt (20), and the pivoting center of said bolt link (26) and said bolt (20) is axially behind the pivoting center of said feeding loop claw (21) and said bolt (20).

6. The actuation structure of the blood vessel clip applier as claimed in claim 1, 2, 3, 4 or 5, wherein said loading plug (17) surrounds said recoil push rod (6).

## Patentansprüche

1. Betätigungsstruktur einer Blutgefäßklemmenanbringungsvorrichtung, die durch eine Betätigungseinheit (200) angetrieben wird, um eine Klemmbacke (1) zum Klemmen einer Klemme (35) zu bringen, worin die Betätigungsstruktur der Blutgefäßklemmenanbringungsvorrichtung umfasst:
einen Körper (100) mit einem Höcker (199);
einen Rohrsatz (700) mit einem hinteren Ende (701), das mit dem Körper (100) verbunden ist, und einem vorderen Ende (702), das die Klemmbacke (1) umfasst;
eine Ladeschubstange (3), die in dem Rohrsatz (700) hin und her beweglich angeordnet ist, um die Klemme (35) zum vorderen Ende des Rohrsatzes (702) vorzuschieben;
einen Bolzen (20), der axial beweglich an dem Körper (100) angeordnet ist;
einen Ladestopfen (17), der in den Körper (100) eingesetzt ist, um die Ladeschubstange (3) zu verbinden;
**dadurch gekennzeichnet, dass** die Betätigungsstruktur ferner umfasst:
eine Zuführschlaufenklaue (21), die an dem Bolzen (20) schwenkbar gelagert ist, worin die Zuführschlaufenklaue (21) ein vorderes Ende (211) und ein hinteres Ende (212) aufweist, die ausgestaltet sind, dass, wenn sich die Zuführschlaufenklaue (21) in Richtung des hinteren Endes des Rohrsatzes (701) vorwärts bewegt, die Zuführschlaufenklaue (21) von dem Höcker (199) des Körpers (100) berührt wird, so dass das vordere Ende (211) der Zuführschlaufenklaue (21) von einer herausgekippten Position in eine heruntergedrückte Position wechselt;
eine Ladefeder (18), um den Ladestopfen (17) nach hinten in Richtung des hinteren Endes des Rohrsatzes (701) zu drücken;
wobei, wenn das vordere Ende (211) der Zuführschlaufenklaue (21) herausgekippt wird, der Ladestopfen (17) in Richtung des vorderen Endes des Rohrsatzes (702) nach vorne geschoben wird; und wobei, wenn das vordere Ende (211) der Zuführschlaufenklaue (21) nach unten gedrückt wird, das vordere Ende (211) von dem Ladestopfen (17) ferngehalten wird, so dass die Ladefeder (18) den Ladestopfen (17) in seine ursprüngliche Position zurückschiebt;
eine Rückstoßschubstange (6), die beweglich in den Rohrsatz (700) eingesetzt ist, so dass, nachdem der Ladestopfen (17) in seine ursprüngliche Position zurückgekehrt ist, das vordere Ende (211) der Zuführschlaufenklaue (21) immer noch nach unten gedrückt wird, um die Rückstoßschubstange (6) nach vorne in Richtung des vorderen Endes des Rohrsatzes (702) zu schieben, um die Klemmbacke (1) zum Festklemmen der Klemme (35) anzutreiben; und
eine Rückstoßfeder (19), die verwendet wird, um die Rückstoßschubstange (6) nach hinten in Richtung des hinteren Endes des Rohrsatzes (701) zu drücken.

2. Betätigungsstruktur der Blutgefäßklemmenanbringungsvorrichtung nach Anspruch 1, worin der Bolzen (20) einen Aufnahmeschlitz (201) aufweist und die Zuführschlaufenklaue (21) in dem Aufnahmeschlitz (201) eingerichtet ist.

3. Betätigungsstruktur der Blutgefäßklemmenanbringungsvorrichtung nach Anspruch 1 oder 2, worin der Körper (100) eine Buchse (110) umfasst und der Ladestopfen (17) in der Buchse (110) eingerichtet ist.

4. Betätigungsstruktur der Blutgefäßklemmenanbringungsvorrichtung nach Anspruch 1, 2 oder 3, welche ferner ein elastisches Teil (28) umfasst, das auf den Bolzen (20) aufgesetzt ist, worin das elastische Teil (28) gegen das hintere Ende (212) der Zuführschlaufenklaue (21) drückbar ist, so dass das vordere Ende (211) der Zuführschlaufenklaue (21) gegen den Ladestopfen (17) herausgekippt wird.

5. Betätigungsstruktur der Blutgefäßklemmenanbringungsvorrichtung nach Anspruch 1, 2, 3 oder 4, worin die Betätigungseinheit (200) eine Bolzenverbindung (26) umfasst, die den Bolzen (20) schwenkt, und das Schwenkzentrum der Bolzenverbindung (26) und des Bolzens (20) axial hinter dem Schwenkzentrum der Zuführschlaufenklaue (21) und des Bolzens (20) liegt.

6. Betätigungsstruktur der Blutgefäßklemmenanbringungsvorrichtung nach Anspruch 1, 2, 3, 4 oder 5, worin der Ladestopfen (17) die Rückstoßschubstange (6) umgibt.

## Revendications

1. Structure d'actionnement d'un l'applicateur de clip de vaisseau sanguin entraîné par une unité de commande (200) pour faire en sorte qu'une mâchoire (1) serre un clip (35), la structure d'actionnement de l'applicateur de clip de vaisseau sanguin comprenant:
un corps (100) comprenant une bosse (199);
un ensemble de tubes (700) comprenant une extrémité arrière (701) reliée au corps (100) et une extrémité avant (702) comprenant la mâchoire (1);
une tige de poussée de chargement (3) placée de façon mobile dans ledit ensemble de tubes (700) d'avant en arrière pour pousser ledit clip (35) vers l'extrémité avant de l'ensemble de tubes (702);
un boulon (20) disposé de manière axialement mobile sur ledit corps (100);
uni fiche de chargement (17) placé dans ledit corps (100) pour relier ladite tige de poussée de chargement (3);
**caractérisée par le fait que** la structure d'actionnement comprend en outre:
une griffe de boucle d'alimentation (21) pivotée sur ledit boulon (20), ladite griffe de boucle d'alimentation (21) comprenant une extrémité avant (211) et une extrémité arrière (212) configurée de telle sorte que lorsque ladite griffe de boucle d'alimentation (21) se déplace vers l'avant en direction de l'extrémité arrière de l'ensemble de tubes (701), ladite griffe de boucle d'alimentation (21) est touchée par ladite bosse (199) dudit corps (100) de telle sorte que l'extrémité avant (211) de ladite griffe de boucle d'alimentation (21) passe d'une position inclinée vers l'extérieur à une position pressée vers le bas;
un ressort de chargement (18) pour pousser ladite fiche de chargement (17) à reculer en direction de l'extrémité arrière de l'ensemble de tubes (701);
Lorsque l'extrémité avant (211) de ladite griffe de boucle d'alimentation (21) est inclinée vers l'extérieur, la fiche de chargement (17) est poussée vers l'avant en direction de l'extrémité avant de l'ensemble de tubes (702); lorsque l'extrémité avant (211) de ladite griffe de boucle d'alimentation (21) est pressée vers le bas, l'extrémité avant (211) est maintenue à distance de la fiche de chargement (17), de sorte que le ressort de chargement (18) repousse la fiche de chargement (17) dans sa position d'origine;
une tige de poussée de recul (6) placée de manière mobile dans ledit ensemble de tubes (700), de sorte qu'après que ladite fiche de chargement (17) soit revenu à sa position initiale, l'extrémité avant (211) de ladite griffe de boucle d'alimentation (21) est encore pressée vers le bas pour pousser ladite tige de poussée de recul (6) vers l'avant en direction de l'extrémité avant de l'ensemble de tubes (702) afin d'entraîner ladite mâchoire (1) qui serre ledit clip (35); et
un ressort de recul (19) utilisé pour pousser ladite tige de poussée de recul (6) vers l'arrière en direction de l'extrémité arrière de l'ensemble de tubes (701).

2. Structure d'actionnement de l'applicateur de clip de vaisseau sanguin selon la revendication 1, dans laquelle ledit boulon (20) comprend une fente de réception (201), et ladite griffe de boucle d'alimentation (21) est installée dans ladite fente de réception (201).

3. Structure d'actionnement de l'applicateur de clip de vaisseau sanguin selon la revendication 1 ou 2, dans laquelle ledit corps (100) comprend une douille (110), et ladite fiche de chargement (17) est installé dans ladite douille (110).

4. Structure d'actionnement de l'applicateur de clip de vaisseau sanguin selon la revendication 1, 2 ou 3, comprend en outre une pièce élastique (28) fixée sur ledit boulon (20), ladite pièce élastique (28) pouvant être pressée contre l'extrémité arrière (212) de ladite griffe de boucle d'alimentation (21) de sorte que l'extrémité avant (211) de ladite griffe de boucle d'alimentation (21) est inclinée vers l'extérieur contre ladite fiche de chargement (17).

5. Structure d'actionnement de l'applicateur de clip de vaisseau sanguin selon la revendication 1, 2, 3 ou 4, dans laquelle ladite unité de commande (200) comprend une liaison de boulon (26) pivotant ledit boulon (20), et le centre de pivotement de ladite liaison de boulon (26) et dudit boulon (20) est axialement derrière le centre de pivotement de ladite griffe de boucle d'alimentation (21) et dudit boulon (20).

6. Structure d'actionnement de l'applicateur de clip de vaisseau sanguin selon la revendication 1, 2, 3, 4 ou 5, dans laquelle ladite fiche de chargement (17) entoure la tige de poussée de recul (6).
